# EUROPEAN PATENT APPLICATION

(11) **EP 4 059 502 A1**
(43) Date of publication of application: **21.09.2022**
(21) Application number: 20887321.6
(22) Date of filing: 11.11.2020
(51) Int. Cl.: A61K 31/496, A61K 9/20, A61K 47/26, A61K 47/36, A61P 25/24, A61P 25/26

(54) **ORALLY DISINTEGRATING TABLET**

(30) Priority: 11.11.2019 WO PCT/JP2019/044118
(71) Applicant: Otsuka Pharmaceutical Co., Ltd., Tokyo 101-8535 (JP)
(72) Inventor: KEMMOCHI, Taku, Osaka-shi, Osaka 540-0021 (JP); YAMAZAKI, Hiroyuki, Osaka-shi, Osaka 540-0021 (JP); OKA, Yoshikazu, Osaka-shi, Osaka 540-0021 (JP); KOSEKI, Mika, Osaka-shi, Osaka 540-0021 (JP)
(74) Representative: Hoffmann Eitle
(86) International application number: PCT/JP2020/042114
(87) International publication number: WO 2021/095779

(57) **Abstract**

Provided is an orally disintegrating tablet comprising brexpiprazole or a salt thereof that rapidly disintegrates in the oral cavity while having hardness suitable for practical use.

More specifically, provided is an orally disintegrating tablet comprising (A) brexpiprazole or a salt thereof, (B) D-mannitol, (C) partially pregelatinized starch, and (D) a lubricant.

## Description

### Technical Field

The present disclosure relates to an orally disintegrating tablet comprising brexpiprazole or a salt thereof, a method for producing the tablet, and the like. The contents of all of the documents mentioned in the present specification are incorporated herein by reference.

### Background Art

7-[4-(4-Benzo[b]thiophen-4-yl-piperazin-1-yl)butoxy]-1H-quinolin-2-one (also referred to as brexpiprazole) or a salt thereof has a dopamine D₂ receptor partial agonistic action, a serotonin 5-HT_{2A} receptor antagonistic action, and an adrenergic α ₁ receptor antagonistic action. In addition to those actions, brexpiprazole or a salt thereof is known to have a serotonin uptake inhibitory action (or a serotonin reuptake inhibitory action) (Patent Literature 1), and has a broad therapeutic spectrum for central nervous system diseases (particularly schizophrenia).

### Citation List

### Patent Literature

PTL 1: JP2006-316052A

### Summary of Invention

### Technical Problem

The present inventors conducted studies for the main purpose of providing a tablet comprising brexpiprazole or a salt thereof that rapidly disintegrates in the oral cavity while having hardness suitable for practical use.

### Solution to Problem

The present inventors found the possibility that an orally disintegrating tablet that rapidly disintegrates in the oral cavity while having hardness suitable for practical use can be prepared by incorporating specific components in addition to brexpiprazole or a salt thereof. The inventors made further improvements.

The present disclosure includes, for example, the subjects described in the following Items.

### Item 1.

An orally disintegrating tablet comprising:
(A) brexpiprazole or a salt thereof;
(B) D-mannitol;
(C) partially pregelatinized starch; and
(D) a lubricant.

### Item 2.

The orally disintegrating tablet according to Item 1, wherein the lubricant (D) comprises magnesium stearate and sodium stearyl fumarate.

### Item 3.

The orally disintegrating tablet according to Item 1, wherein the lubricant (D) comprises (D1) an internal lubricant and (D2) an external lubricant.

### Item 4.

The orally disintegrating tablet according to Item 3, wherein the internal lubricant (D1) comprises sodium stearyl fumarate, and the external lubricant (D2) comprises magnesium stearate.

### Item 5.

The orally disintegrating tablet according to any one of Items 1 to 4, wherein the D-mannitol (B) has a 50% particle diameter of 10 µm to 100 um, and the D-mannitol (B) is present in non-needle-like crystalline form in the tablet.

### Item 6.

The orally disintegrating tablet according to any one of Items 1 to 5, wherein the partially pregelatinized starch (C) is partially pregelatinized starch having a water-soluble component content of 10% or less.

### Item 7.

The orally disintegrating tablet according to any one of Items 1 to 6, further comprising (E) crystalline cellulose.

### Item 8.

The orally disintegrating tablet according to Item 7, comprising the crystalline cellulose (E) in an amount of 5 to 15 wt%.

### Item 9.

The orally disintegrating tablet according to any one of Items 1 to 8, further comprising (F) low-substituted hydroxypropyl cellulose.

### Item 10.

The orally disintegrating tablet according to any one of Items 1 to 9, which has a tablet hardness of 15 N to 70 N in a diametrical direction thereof, as measured with a tablet hardness tester, and a disintegration time of 70 seconds or less, as measured by the test for immediate-release preparations (plain tablets) described in 6.09 Disintegration Test of the General Tests, Processes and Apparatus section of the Japanese Pharmacopoeia.

### Item 11.

The orally disintegrating tablet according to any one of Items 1 to 10, which is for preventing or treating a central nervous system disease.

### Item 12.

The orally disintegrating tablet according to Item 11, which is for preventing or treating a central nervous system disease selected from the group consisting of schizophrenia, treatment-resistant, refractory, or chronic schizophrenia, emotional disturbance, psychotic disorder, mood disorder, bipolar disorder, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, cognitive impairment, cognitive impairment associated with neurodegenerative disease, cognitive impairment caused by neurodegenerative disease, cognitive impairment in schizophrenia, cognitive impairment caused by treatment-resistant, refractory, or chronic schizophrenia, vomiting, motion sickness, obesity, migraine, pain, mental retardation, autistic disorder, Tourette's disorder, tic disorder, attention deficit hyperactivity disorder, conduct disorder, Down's syndrome, impulsive symptoms associated with dementia, and borderline personality disorder.

### Item 13.

An orally disintegrating tablet comprising:
(A) brexpiprazole or a salt thereof;
(B) D-mannitol;
(C) partially pregelatinized starch; and
(D) a lubricant,
the orally disintegrating tablet being produced by external lubrication method for compression.

### Item 14.

A method for producing an orally disintegrating tablet comprising (A) brexpiprazole or a salt thereof, (B) D-mannitol, (C) partially pregelatinized starch, and (D) a lubricant, the lubricant (D) comprising (D1) an internal lubricant and (D2) an external lubricant, the method comprising the steps of:
mixing (A) brexpiprazole or a salt thereof, (B) D-mannitol, (C) partially pregelatinized starch, and (D1) an internal lubricant; and
compressing the mixture,
wherein in the compression step, (D2) an external lubricant is added by spraying.

### Item A-1.

An orally disintegrating tablet comprising:
(A) brexpiprazole or a salt thereof;
(B) D-mannitol;
(C) partially pregelatinized starch; and
(D) a lubricant,
wherein the brexpiprazole or a salt thereof (A), the D-mannitol (B), and the partially pregelatinized starch (C) are granulated by wet granulation.

### Item A-2.

A method for producing an orally disintegrating tablet comprising (A) brexpiprazole or a salt thereof, (B) D-mannitol, (C) partially pregelatinized starch, and (D) a lubricant, the method comprising the steps of:
granulating (A) brexpiprazole or a salt thereof, (B) D-mannitol, and (C) partially pregelatinized starch by wet granulation;
further mixing (D) a lubricant with the granulated mixture; and
compressing the obtained mixture.

### Item A-3.

An orally disintegrating tablet comprising or preferably consisting of:
brexpiprazole;
D-mannitol;
partially pregelatinized starch;
sodium stearyl fumarate and magnesium stearate;
crystalline cellulose;
partially pregelatinized starch;
sucralose;
at least one coloring agent selected from the group consisting of red ferric oxide, yellow ferric oxide, and blue no. 2 aluminum lake; and
corn starch.

### Advantageous Effects of Invention

An orally disintegrating tablet comprising brexpiprazole or a salt thereof that rapidly disintegrates in the oral cavity while having hardness suitable for practical use is provided.

### Brief Description of Drawings

Fig. 1 shows the disintegration time of the orally disintegrating tablets of Examples 1-1 to 1-3.
Fig. 2a shows the hardness retention of the orally disintegrating tablets of Examples 2-1 to 2-3.
Fig. 2b shows the disintegration time of the orally disintegrating tablets of Examples 2-1 to 2-3.

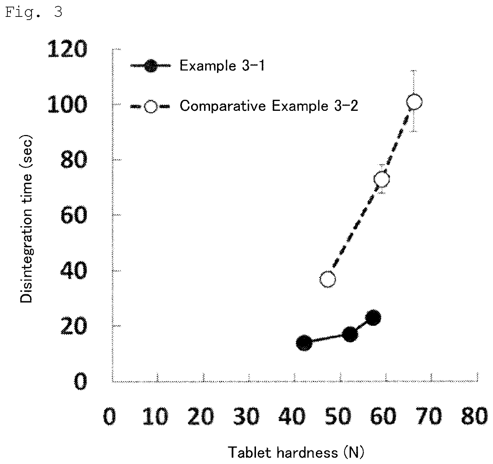

Fig. 3 shows the relationship between the disintegration time and the tablet hardness of the orally disintegrating tablets of Example 3-1 and Comparative Example 3-2.
Fig. 4a shows the hardness of the orally disintegrating tablets of Examples 4-1 to 4-4.
Fig. 4b shows the disintegration time of the orally disintegrating tablets of Examples 4-1 to 4-4.
Fig. 5 shows the relationship between the disintegration time and the tablet hardness of the orally disintegrating tablets of Examples 4-2, 4-4, and 5-3.
Fig. 6a shows the dissolution property of the orally disintegrating tablets of Examples 6-1 to 6-3.
Fig. 6b shows the tablet property (relationship between the hardness and the disintegration property) of the orally disintegrating tablets of Examples 6-1 to 6-3.
Fig. 7a shows electron micrographs before and after granulation in the production of the orally disintegrating tablets of Examples 7-1 and 7-2, and electron micrographs of the cross-sections of the produced orally disintegrating tablets.
Fig. 7b shows photographs of the surface of a punch of a rotary tablet press machine in the production of the orally disintegrating tablet of Example 7-1, and the appearance of the orally disintegrating tablet.

### Description of Embodiments

Embodiments included in the present disclosure are described in more detail below. The present disclosure preferably includes an orally disintegrating tablet comprising brexpiprazole or a salt thereof, a method for producing the orally disintegrating tablet, and the like. However, the present disclosure is not limited thereto, and includes everything that is disclosed in the present specification and that can be recognized by one skilled in the art.

The orally disintegrating tablet included in the present disclosure comprises, in addition to (A) brexpiprazole or a salt thereof, (B) D-mannitol, (C) partially pregelatinized starch, and (D) a lubricant. The orally disintegrating tablet included in the present disclosure may referred to as "the orally disintegrating tablet according to the present disclosure."

### Brexpiprazole or Salt Thereof

The salt of brexpiprazole is not particularly limited as long as it is pharmacologically acceptable. Examples include metal salts such as alkali metal salts (e.g., sodium salts and potassium salts) and alkaline earth metal salts (e.g., calcium salts and magnesium salts), ammonium salts, alkali metal carbonates (e.g., lithium carbonate, potassium carbonate, sodium carbonate, and cesium carbonate), alkali metal hydrogen carbonates (e.g., lithium hydrogen carbonate, sodium hydrogen carbonate, and potassium hydrogen carbonate), alkali metal hydroxides (e.g., lithium hydroxide, sodium hydroxide, potassium hydroxide, and cesium hydroxide), and like inorganic base salts; tri(lower)alkylamines (e.g., trimethylamine, triethylamine, N-ethyldiisopropylamine), pyridine, quinoline, piperidine, imidazole, picoline, dimethylaminopyridine, dimethylaniline, N-(lower)alkyl-morpholines (e.g., N-methylmorpholine), 1,5-diazabicyclo[4.3.0]nonene-5 (DBN), 1,8-diazabicyclo[5.4.0]undecene-7 (DBU), 1,4-diazabicyclo[2.2.2]octane (DABCO), and like organic base salts; hydrochloric acid salts, hydrobromic acid salts, hydriodic acid salts, sulfuric acid salts, nitric acid salts, phosphoric acid salts, and like inorganic acid salts; formic acid salts, acetic acid salts, propionic acid salts, oxalic acid salts, malonic acid salts, succinic acid salts, fumaric acid salts, maleic acid salts, lactic acid salts, malic acid salts, citric acid salts, tartaric acid salts, carbonic acid salts, picric acid salts, methanesulfonic acid salts, ethanesulfonic acid salts, p-toluenesulfonic acid salts, glutamic acid salts, and like organic acid salts; and the like.

Although there is no particular limitation, the orally disintegrating tablet according to the present disclosure may comprise the brexpiprazole or a salt thereof (A) in an amount of, for example, about 0.1 to 10 wt% or about 0.3 to 5 wt%. Although there is no particular limitation, the brexpiprazole or a salt thereof (A) may be contained in an amount of, for example, about 0.05 to 10 mg, about 0.1 to 8 mg, or about 0.5 to 5 mg per orally disintegrating tablet according to the present disclosure.

### D-Mannitol

In one embodiment, although there is no particular limitation, the D-mannitol (B) used in the orally disintegrating tablet according to the present disclosure may have a 50% particle diameter of, for example, 10 µm to 100 µm, preferably 15 µm to 80 µm, and more preferably 20 µm to 50 µm. The 50% particle diameter, also called D50 or the median diameter, refers to the particle diameter in which 50% of the particles have a diameter greater than this value, and 50% of the particles have a diameter less than this value. The 50% particle diameter is determined by a laser diffraction scattering method.

The D-mannitol (B) is known to exist in polymorphs such as alpha (α), beta (β), and delta (δ) and have different crystal shapes such as plate-like, needle-like, and porous shapes. It is generally known that the crystal shape (crystalline form, crystal habit) is determined by the ratio of the growth rate of each crystal face, and changes significantly under the influence of crystal growth inhibitor and impurities in the solution. For example, if the growth of the sides of a crystal is inhibited due to some cause, the crystal grows in only one direction and takes a needle-like form. Various other crystal shapes have been reported, including plate-like, prismatic, cubic, dendritic, and bulky shapes (Masakuni Matsuoka (2010). Base & Application of Polymorphic Crystals. Trade ed, CMC Publishing Co., Ltd.; Hiroshi Takiyama (2013), Shoseki no Kyokasho (Textbook for Crystallization). First ed., S&T Publishing Inc.). Commercially available D-mannitol in beta crystalline form is generally produced as a plate-like crystalline powder. Commercially available D-mannitol in delta crystalline form undergoes a transition from delta form to beta form during granulation, in which the crystal shape changes from a plate-like shape to a needle-like shape. The crystal shape of D-mannitol in tablets and granules can be determined by observation using a microscope, for example. D-mannitol in any crystal polymorph may be used in the orally disintegrating tablet according to the present disclosure, and it is preferable to use D-mannitol in beta form. In the production of the orally disintegrating tablet, the use of D-mannitol present in a non-needle-like, preferably plate-like crystalline form during tableting is particularly effective in suppressing the filming phenomenon described later, and is thus preferred. Thus, in one embodiment, the D-mannitol (B) in the orally disintegrating tablet according to the present disclosure is present in a non-needle-like, preferably plate-like, crystalline form in the tablet.

In one embodiment, the D-mannitol (B) in the orally disintegrating tablet according to the present disclosure has a 50% particle diameter of 10 µm to 100 µm and is present in a non-needle-like, preferably plate-like, crystalline form in the tablet.

Although there is no particular limitation, the orally disintegrating tablet according to the present disclosure may comprise the D-mannitol (B) in an amount of, for example, about 20 to 90 wt%, about 40 to 85 wt%, about 55 to 85 wt%, or about 60 to 80 wt%. Further, although there is no particular limitation, the D-mannitol (B) may be present in an amount of, for example, about 10 to 180 parts by weight, about 20 to 160 parts by weight, or about 30 to 140 parts by weight, per part by weight of the brexpiprazole or a salt thereof (A).

### Partially Pregelatinized Starch

The partially pregelatinized starch (C) refers to partially pregelatinized starch that swells and becomes a white turbid liquid when water is added thereto. On the other hand, pregelatinized starch refers to one that becomes a viscous paste-like liquid when water is added thereto. The partially pregelatinized starch can be prepared by heating starch (preferably corn starch) with water under ordinary pressure or increased pressure (and, if necessary, performing drying). The partially pregelatinized starch preferably has a water-soluble component content of 10% or less, and more preferably 9% or less, 8% or less, 7% or less, 6% or less, 5% or less, 4% or less, or 3% or less. The amount of water-soluble component in the partially pregelatinized starch is measured by the test method described in "Cold-water-soluble matter" in the "STARCH, PREGELATINISED" section of European Pharmacopoeia 9.0.

The degree of pregelatinization of the partially pregelatinized starch (C) in the orally disintegrating tablet according to the present disclosure is preferably 70% or less, and more preferably 30% to 70%, 40% to 70%, or 50% to 70%. The degree of pregelatinization of the partially pregelatinized starch is the state of gelatinization (pregelatinization) in starch expressed numerically as a percentage, and is measured by, for example, a glucoamylase method.

Although there is no particular limitation, the orally disintegrating tablet according to the present disclosure may comprise the partially pregelatinized starch (C) in an amount of, for example, about 1 to 15 wt%, about 1.5 to 10 wt%, or about 2 to 8 wt%. Further, although there is no particular limitation, the partially pregelatinized starch (C) may be present in an amount of, for example, about 0.5 to 30 parts by weight, about 1 to 20 parts by weight, or about 1 to 15 parts by weight, per part by weight of the brexpiprazole or a salt thereof (A).

### Lubricant

Examples of the lubricant (D) include stearic acid or salts thereof (e.g., aluminum stearate, calcium stearate, and magnesium stearate), carnauba wax, glycerin fatty acid esters, hydrogenated oil, beeswax, white beeswax, talc, fumaric acid, sodium stearyl fumarate, polyethylene glycol (macrogols, such as macrogol 400, macrogol 600, macrogol 1500, macrogol 4000, and macrogol 6000), and the like. These lubricants may be used singly, or in a combination of two or more. Among these, stearic acid salts, sodium stearyl fumarate, sucrose fatty acid esters, and hydrogenated oil are preferable, and magnesium stearate and sodium stearyl fumarate are more preferable. It is particularly preferable to use magnesium stearate and sodium stearyl fumarate in combination.

As the lubricant (D), there are (D1) an internal lubricant contained in the inner portion of the tablet and (D2) an external lubricant contained in the outer portion of the tablet, as described later. The orally disintegrating tablet according to the present disclosure preferably comprises both the internal lubricant (D1) and the external lubricant (D2) as the lubricant (D). The "outer portion of the tablet" refers to the surface of the tablet, more specifically, the portion up to 0.1 mm from the surface of the tablet. When the orally disintegrating tablet according to the present disclosure comprises an internal lubricant and an external lubricant, the internal lubricant and the external lubricant may be the same component or different components. As the internal lubricant, a single component or a combination of two or more components may be used. As the external lubricant, a single component or a combination of two or more components may be used.

The orally disintegrating tablet according to the present disclosure preferably comprises sodium stearyl fumarate as the internal lubricant (D1), and magnesium stearate as the external lubricant (D2).

Although there is no particular limitation, the orally disintegrating tablet according to the present disclosure may comprise the lubricant (D) in an amount of, for example, about 0.1 to 5 wt%, about 0.2 to 3 wt%, or about 0.3 to 2 wt%. Further, although there is no particular limitation, the lubricant (D) may be present in an amount of, for example, about 0.05 to 2 parts by weight or about 0.1 to 1.5 parts by weight, per part by weight of the brexpiprazole or a salt thereof (A).

The orally disintegrating tablet according to the present disclosure may comprise other components in addition to the above components (A) to (D).

### Crystalline Cellulose

For example, the orally disintegrating tablet according to the present disclosure may comprise (E) crystalline cellulose. The crystalline cellulose is not particularly limited. For example, the crystalline cellulose preferably has an average particle diameter of about 10 to 100 µm, about 20 to 80 µm, about 30 to 70 µm, or about 40 to 60 µm. Further, for example, the crystalline cellulose preferably has a bulk density of about 0.1 to 0.5 g/cm³, about 0.15 to 0.45 g/cm³, about 0.2 to 0.4 g/cm³, or about 0.25 to 0.35 g/cm³.

The average particle diameter and the bulk density are values obtained by measurement according to the General Tests, Processes and Apparatus section (3.01 Determination of Bulk and Tapped Densities, and 3.04 Particle Size Determination) of the Japanese Pharmacopoeia, Seventeenth Edition. More specifically, a method using a volumeter, which is Method 2, is used for measurement of the bulk density; and mechanical agitation, which is a sieving method, is used for particle size determination.

The orally disintegrating tablet according to the present disclosure may comprise the crystalline cellulose (E) in an amount of, for example, about 1 to 20 wt%, about 5 to 15 wt%, or about 7.5 to 12.5 wt%. Further, the crystalline cellulose (E) may be present in an amount of, for example, about 0.1 to 40 parts by weight, about 1 to 30 parts by weight, or about 2 to 25 parts by weight, per part by weight of the brexpiprazole or a salt thereof (A).

### Low-substituted Hydroxypropyl Cellulose

For example, the orally disintegrating tablet according to the present disclosure may also comprise (F) low-substituted hydroxypropyl cellulose. The low-substituted hydroxypropyl cellulose is preferably hydroxypropyl cellulose having a hydroxypropoxy group content of about 5 to 16 (mass)%. The upper or lower limit of the range may be about 6, 7, 8, 9, 10, 11, 12, 13, 14, or 15 (mass)%. The hydroxypropoxy group content of the low-substituted hydroxypropyl cellulose can be measured by a method described in the Japanese Pharmacopoeia, Seventeenth Edition. The low-substituted hydroxypropyl cellulose may be produced by a known production method, or may be a commercial available product. Examples of commercial available products of the low-substituted hydroxypropyl cellulose include, but are not limited to, "LH series" and "NBD series" produced by Shin-Etsu Chemical Co., Ltd., and the like.

The orally disintegrating tablet according to the present disclosure may comprise the low-substituted hydroxypropyl cellulose (F) in an amount of, for example, about 1 to 20 wt%, about 2 to 15 wt%, or about 2 to 10 wt%. Further, the low-substituted hydroxypropyl cellulose (D) may be present in an amount of, for example, about 0.5 to 40 parts by weight, about 1 to 30 parts by weight, or about 2 to 25 parts by weight, per part by weight of the brexpiprazole or a salt thereof (A).

### Other Additives

For example, the orally disintegrating tablet according to the present disclosure may also comprise components that are other than the above and that are known in the field of pharmaceutical tablets, as long as the effects of the orally disintegrating tablet according to the present disclosure are not impaired. Examples of such components include excipients, binders, disintegrators, coloring agents, pH adjusters, preservatives, absorption promoters, taste enhancers, antioxidants, buffers, chelating agents, abrasives, solvents, hardening agents, surfactants, sweeteners, fluidizers, brightening agents, flavors, and the like. These other components may be used singly, or in a combination of two or more.

Specific examples of excipients include sugar such as fructose, white soft sugar, sucrose, powdered sugar, lactose, powdered hydrogenated maltose starch syrup, and maltose; sugar alcohols such as D-sorbitol, xylitol, erythritol, and maltitol; starch such as wheat starch, corn starch, and potato starch; starch derivatives such as dextrin and β-cyclodextrin; cellulose or a derivative thereof such as ethyl cellulose, carboxymethyl cellulose (carmellose), and sodium carboxymethyl cellulose (carmellose sodium); silicic acid or a salt thereof such as light anhydrous silicic acid, hydrated silicon dioxide, silicon dioxide, calcium silicate, magnesium silicate, and magnesium aluminometasilicate; kaolin; titanium oxide; magnesium oxide; talc; precipitated calcium carbonate; anhydrous dibasic calcium phosphate; and the like. These excipients may be used singly, or in a combination of two or more. Examples of binders include pregelatinized starch; cellulose or a derivative thereof such as hydroxyethyl cellulose, hydroxyethyl methyl cellulose, hydroxypropyl cellulose, and hydroxypropyl methyl cellulose; other polysaccharides such as gum arabic, powdered gum arabic, agar, powdered agar, guar gum, tragacanth, powdered tragacanth, pullulan, and pectin; acrylic acid-based polymers such as methacrylic acid copolymer L, methacrylic acid copolymer LD, methacrylic acid copolymer S, ethyl acrylate-methyl methacrylate copolymer dispersion, aminoalkyl methacrylate copolymer E, and aminoalkyl methacrylate copolymer RS; sodium alginate; purified gelatin; hydrolyzed gelatin powder; carboxyvinyl polymer; copolyvidone; povidone; polyvinyl alcohol; and the like. These binders may be used singly, or in a combination of two or more. Examples of sweeteners include aspartame, sucralose, and the like. Examples of coloring agents include red ferric oxide, yellow ferric oxide, blue no. 2 aluminum lake, and the like. These may be used singly, or in a combination of two or more.

### Production Method

The present disclosure also relates to a method for producing the orally disintegrating tablet. The orally disintegrating tablet according to the present disclosure can be produced, for example, by the steps of preparing a mixture containing the components (A) to (D) (if necessary, the component (E) and/or the component (F) may also be used, and further other components may also be used) and forming the mixture into tablets (e.g., compressing the mixture).

In one embodiment, the step of preparing a mixture containing the components (A) to (D) (and, if necessary, the component (E), the component (F), and/or other components) may be performed by granulating a mixture containing the components (A) to (C), and further mixing the lubricant (D) therewith. In the above step, the component (E), the component (F), and/or other components may be added at any suitable stage as necessary.

The granulation method is not particularly limited. Examples thereof include dry granulation methods, wet granulation methods (e.g., a fluidized-bed granulation method and a knead-granulation method); and the like. Among these, wet granulation methods (in particular, a fluidized-bed granulation method) are preferably used for the production from the viewpoint of being able to uniformly mix the active ingredient and other components, and being able to obtain a tablet whose components are uniformly distributed therein. Thus, in one embodiment, the method for producing the orally disintegrating tablet according to the present disclosure comprises the step of granulating a mixture containing the components (A) to (C) (and, if necessary, the component (E), the component (F), and/or other components) by wet granulation. In one embodiment, the orally disintegrating tablet according to the present disclosure comprises the components (A) to (C) granulated by wet granulation. In other words, the orally disintegrating tablet according to the present disclosure may be preferably prepared using particles containing the components (A) to (C) produced by wet granulation.

Examples of the tablet forming method include tableting, such as direct compression tableting, dry tableting, wet tableting, external lubrication method for compression, and the like.

Continuous production of tablets by tableting may cause filming or adhesion to the turntable in a rotary tablet press machine. Filming is a phenomenon in which powder adheres to the surface of the punches of a rotary tablet press machine. When this happens, the tableting is performed with the punches having the powder adhered thereto; thus, the tables produced have a rough, dull (coarse) surface. As a result, in printing, ink permeates, leading to problems such as unclear printing due to ink bleeding and rubbing, and illegible imprints such as corporate symbols and product codes. In addition, tablets with a rough surface often wear due to impact during the distribution process, even if they are sorted as normal products. Further, when such tablets are pushed out of a blister sheet or placed in the cassette of a fully-automatic tablet packaging machine, worn powder adheres to the tablets, which is undesirable. Moreover, if the tableting process is continued with filming occurring, the powder itself adhered to the punches agglomerates. If tablets are produced by tableting with such punches to which agglomerated powder adheres, a phenomenon called sticking occurs in which a recess is formed on the tablet surface, or in which the imprint is missing. Thus, each time filming occurs, the powder adhered to the punches should be removed, which is bothersome. Adhesion to the turntable is a phenomenon in which powder adheres to the turntable (the area where dies are set) of a rotary tablet press machine. In this case, the likelihood of adverse effects on the resulting tablet itself is smaller than in filming; however, it is also preferable to remove the adhered powder, which is bothersome. Accordingly, although filming or adhesion to the turntable itself does not pose a problem for the therapeutic efficacy of the orally disintegrating tablet according to the present disclosure, it is preferable to use a production method that does not cause filming or adhesion to the turntable (especially filming) from the viewpoint of handling by health professionals, ease of use for patients, and production efficiency.

In the production of the orally disintegrating tablet according to the present disclosure, the use of external lubrication method for compression is particularly preferable because it suppresses filming. Therefore, in one embodiment, the orally disintegrating tablet according to the present disclosure is produced by external lubrication method for compression. More specifically, it is preferable to add a lubricant by spraying in the step of forming the above-mentioned mixture into tablets (compressing the mixture) (i.e., during compressing). In particular, it is preferable that a lubricant is also contained in the mixture. In this embodiment, the lubricant contained in the composition (preferably mixture) to be compressed may be the internal lubricant (D1), and the lubricant to be added by spraying during compressing may be the external lubricant (D2).

Thus, in one embodiment, the orally disintegrating tablet according to the present disclosure can be produced by a method comprising the step of mixing the components (A) to (C) and the internal lubricant (D1) and the step of compressing the mixture, wherein in the compressing step, the external lubricant (D2) is added by spraying (external lubrication method for compression).

Further, in the production method according to the present disclosure, the above-mentioned granulation method and tableting method can be combined. For example, it is more preferable that the orally disintegrating tablet according to the present disclosure is produced by a method comprising the step of granulating a mixture containing the components (A) to (C) (if necessary, the component (E) and/or the component (F) may also be used, and further other components may also be used) and further mixing the internal lubricant (D1) therewith and the step of compressing the obtained mixture, wherein in the compressing step, the external lubricant (D2) is further added by spraying (external lubrication method for compression).

When the tablet is prepared by such external lubrication method for compression, the internal lubricant (D1) contained in the mixture and the external lubricant (D2) added by spraying during compressing may be the same or different. The sum of the weight of the component (D1) and the weight of the component (D2) is the weight of the lubricant (D) contained in the orally disintegrating tablet. The weight ratio of the component (D1) to the component (D2) (D1:D2) is, for example, about 10:0.5 to 15, about 10:1 to 10, or about 10:2 to 9. The weight ratio of the component (D1) to the component (D2) (D1:D2) may also be, for example, about 10:1 to 7, about 10:1 to 6, about 10:1 to 5, or about 10:2 to 5.

Although there is no particular limitation, the internal lubricant (D1) is preferably sodium stearyl fumarate, and the external lubricant (D2) is preferably magnesium stearate and/or sodium stearyl fumarate. More preferably, the internal lubricant (D1) is sodium stearyl fumarate, and the external lubricant (D2) is magnesium stearate, in the orally disintegrating tablet according to the present disclosure.

### Dosage Regimen

The dose of the orally disintegrating tablet according to the present disclosure is suitably selected, for example, according to the intended use; the patient's age, sex, and other conditions; the severity of the disease; and the like. For example, the dose may be selected so that the amount of the brexpiprazole or a salt thereof (A), which is the active ingredient, is about 0.05 to 6 mg per day calculated as brexpiprazole.

### Tablet Hardness

Although there is no particular limitation, the orally disintegrating tablet according to the present disclosure may have a tablet hardness of about 15 to 70 N, about 20 to 60 N, or about 30 to 60 N. Moreover, the orally disintegrating tablet according to the present disclosure preferably has a disintegration time of 70 seconds or less, and more preferably 65 seconds or less, 60 seconds or less, 55 seconds or less, 50 seconds or less, 45 seconds or less, 40 seconds or less, 35 seconds or less, 30 seconds or less, 25 seconds or less, or 20 seconds or less.

The tablet hardness is a value obtained by measuring the hardness in the diametrical direction of the tablet with a tablet hardness tester (e.g., MultiTest 50 (Pharmatron)). The disintegration time is a value obtained by measurement by the test (temperature setting: 37°C±2.0°C, test liquid: water) for immediate-release preparations (plain tablets) described in 6.09 Disintegration Test of the General Tests, Processes and Apparatus section of the Japanese Pharmacopoeia, Seventeenth Edition. For example, a disintegration tester NT-200 (Toyama Sangyo Co., Ltd.) can be used for this measurement.

### Indication

The orally disintegrating tablet according to the present disclosure can be used, for example, for preventing or treating a central nervous system disease.

Specific examples of central nervous system diseases to be prevented or treated using the orally disintegrating tablet according to the present disclosure include, but are not limited to, various central nervous system disorders such as schizophrenia, treatment-resistant, refractory, or chronic schizophrenia, emotional disturbance, psychotic disorder, mood disorder, bipolar disorder (e.g., bipolar I disorder and bipolar II disorder), depression, endogenous depression, major depression, melancholic and treatment-resistant depression, dysthymic disorder, cyclothymic disorder, anxiety disorder (e.g., panic attack, panic disorder, agoraphobia, social phobia, obsessive-compulsive disorder, post-traumatic stress disorder, generalized anxiety disorder, and acute stress disorder), somatoform disorder (e.g., hysteria, somatization disorder, conversion disorder, pain disorder, and hypochondria), factitious disorder, dissociative disorder, sexual disorder (e.g., sexual dysfunction, libido disorder, sexual arousal disorder, and erectile dysfunction), eating disorder (e.g., anorexia nervosa and bulimia nervosa), sleep disorder, adjustment disorder, substance-related disorder (e.g., alcohol abuse, alcohol intoxication, drug addiction, amphetamine addiction, and narcotism), anhedonia (e.g., loss of pleasure, anhedonia, iatrogenic anhedonia, anhedonia of a psychic or mental cause, anhedonia associated with depression, and anhedonia associated with schizophrenia), delirium, cognitive impairment, cognitive impairment associated with Alzheimer's disease, Parkinson's disease, and other neurodegenerative diseases, cognitive impairment caused by Alzheimer's disease, Parkinson's disease, and other neurodegenerative diseases, cognitive impairment in schizophrenia, cognitive impairment caused by treatment-resistant, refractory, or chronic schizophrenia, vomiting, motion sickness, obesity, migraine, pain, mental retardation, autistic disorder (autism), Tourette's disorder, tic disorder, attention deficit hyperactivity disorder, conduct disorder, Down's syndrome, impulsive symptoms associated with dementia (e.g., agitation associated with dementia of the Alzheimer type), and borderline personality disorder.

In this specification, the term "comprising" includes "consisting essentially of" and "consisting of." Further, the present disclosure includes any and all combinations of the components described herein.

Various characteristics (properties, structures, functions, etc.) described in the above embodiments of the present disclosure may be combined in any manner to specify the subject matter included in the present disclosure. That is, this disclosure includes all of the subject matter comprising any combination of the combinable properties described herein.

### Examples

The embodiments of the present disclosure are described in more detail below with reference to Examples. However, the embodiments of the present disclosure are not limited to these Examples.

Unless otherwise specified, brexpiprazole was synthesized according to a known method and then pulverized with a hammer mill for use.

A disintegration test was performed according to the test (temperature setting: 37°C±2.0°C, test liquid: water) for immediate-release preparations (plain tablets) described in 6.09 Disintegration Test of the General Tests, Processes and Apparatus section of the Japanese Pharmacopoeia, Seventeenth Edition. NT-200 (Toyama Sangyo Co., Ltd.) was used as a disintegration tester.

A dissolution test was performed according to 6.10 Dissolution Test of the General Tests, Processes and Apparatus section of the Japanese Pharmacopoeia, Seventeenth Edition (dissolution test liquid: 1st fluid for dissolution test (fluid prepared by dissolving 2.0 g of sodium chloride in 7.0 mL of hydrochloric acid and water to make 1000 mL; pH: about 1.2); paddle rotation speed: 50 rpm; amount of test liquid: 900 mL; temperature setting: 37±0.5°C; measurement wavelength: λ1=214nm, λ2=380nm). NTR-6200A (Toyama Sangyo Co., Ltd.) was used as a dissolution tester.

The hardness of the tablet was determined by measuring the hardness in the diametrical direction with a tablet hardness tester MultiTest 50 (Pharmatron).

### Investigation 1 of Disintegrator

### Example 1-1

According to the formulation shown in Table 1, a suspension of partially pregelatinized starch as a binder was added by spraying to brexpiprazole, D-mannitol, crystalline cellulose, corn starch, low-substituted hydroxypropyl cellulose (LH-11), and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

### Example 1-2

According to the formulation shown in Table 1, a suspension of partially pregelatinized starch as a binder was added by spraying to brexpiprazole, D-mannitol, crystalline cellulose, corn starch, sodium starch glycolate (Primojel), and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

### Example 1-3

According to the formulation shown in Table 1, a suspension of partially pregelatinized starch as a binder was added by spraying to brexpiprazole, D-mannitol, crystalline cellulose, corn starch, croscarmellose sodium (Kicolate ND-2HS), and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

**Table 1**

| | Example 1-1 | Example 1-2 | Example 1-3 |
|---|---|---|---|
| Brexpiprazole | 2.0 mg | 2.0 mg | 2.0 mg |
| D-Mannitol | 61.1 mg | 61.1 mg | 61.1 mg |
| Crystalline cellulose (Ceolus PH-101) | 5 mg | 5 mg | 5 mg |
| Corn starch | 10 mg | 10 mg | 10 mg |
| Low-substituted hydroxypropyl cellulose (LH-11) | 5 mg | - | - |
| Sodium starch glycolate (Primojel) | - | 5 mg | - |
| Croscarmellose sodium (Kicolate ND-2HS) | - | - | 5 mg |
| Sucralose | 0.1 mg | 0.1 mg | 0.1 mg |
| Partially pregelatinized starch (PCS PC-10) | 5 mg | 5 mg | 5 mg |
| Sodium stearyl fumarate | 1.8 mg | 1.8 mg | 1.8 mg |
| Total solid component amount | 90.0 mg | 90.0 mg | 90.0 mg |

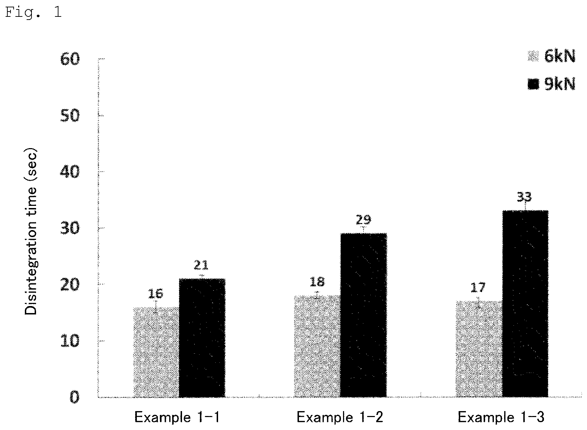

Immediately after the tableting process, each orally disintegrating tablet was subjected to a disintegration test. Fig. 1 shows the results. No impractical disintegration delay was observed when any of the disintegrators was used. Among them, low-substituted hydroxypropyl cellulose was found to be preferable.

### Investigation of Hardness Retention

### Example 2-1

According to the formulation shown in Table 2, a suspension of partially pregelatinized starch as a binder was added by spraying to brexpiprazole, D-mannitol, low-substituted hydroxypropyl cellulose, and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

### Example 2-2

According to the formulation shown in Table 2, a suspension of partially pregelatinized starch as a binder was added by spraying to brexpiprazole, D-mannitol, crystalline cellulose (Ceolus PH-101; 5 mg), low-substituted hydroxypropyl cellulose, and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

### Example 2-3

According to the formulation shown in Table 2, a suspension of partially pregelatinized starch as a binder was added by spraying to brexpiprazole, D-mannitol, crystalline cellulose (Ceolus PH-101; 10 mg), low-substituted hydroxypropyl cellulose, and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 6 kN or 9 kN) to obtain orally disintegrating tablets.

**Table 2**

| | Example 2-1 | Example 2-2 | Example 2-3 |
|---|---|---|---|
| Brexpiprazole | 2.0 mg | 2.0 mg | 2.0 mg |
| D-Mannitol | 76.1 mg | 71.1 mg | 66.1 mg |
| Crystalline cellulose (Ceolus PH-101) | - | 5 mg | 10 mg |
| Low-substituted hydroxypropyl cellulose (NBD-022) | 5 mg | 5 mg | 5 mg |
| Sucralose | 0.1 mg | 0.1 mg | 0.1 mg |
| Partially pregelatinized starch (PCS PC-10) | 5 mg | 5 mg | 5 mg |
| Sodium stearyl fumarate | 1.8 mg | 1.8 mg | 1.8 mg |
| Total solid component amount | 90.0 mg | 90.0 mg | 90.0 mg |

The hardness and disintegration time of each orally disintegrating tablet at the start and stored for 3, 7, and 14 days were measured under humidified storage conditions at 25°C/75% RH (relative humidity).

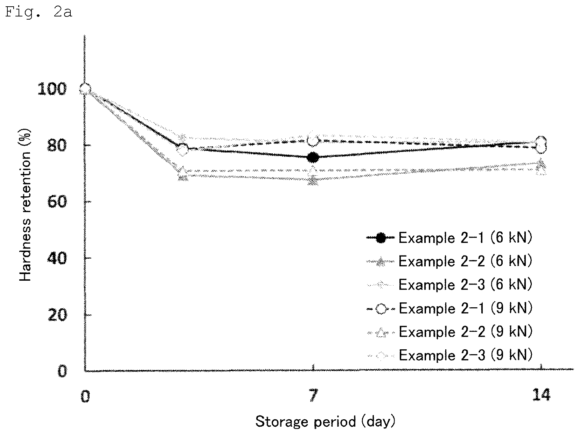

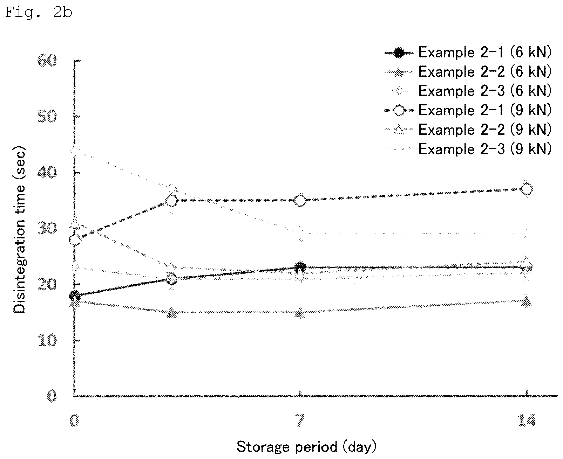

Fig. 2a shows the hardness retention obtained from the hardness measurement results. Fig. 2b shows the disintegration time measurement results. The hardness suitable for practical use was retained for each amount of crystalline cellulose used. Among them, the example using 10 mg of crystalline cellulose (Example 2-3) was found to be preferable.

### Investigation of Binder

### Example 3-1

According to the formulation shown in Table 3, a suspension of partially pregelatinized starch (PCS PC-10) as a binder was added by spraying to brexpiprazole, D-mannitol, crystalline cellulose, low-substituted hydroxypropyl cellulose, and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 4 kN, 5 kN, or 6 kN) to obtain orally disintegrating tablets.

### Comparative Example 3-2

According to the formulation shown in Table 3, a suspension of pregelatinized starch (SWELSTAR WB-1) as a binder was added by spraying to brexpiprazole, D-mannitol, crystalline cellulose, low-substituted hydroxypropyl cellulose, and sucralose, which are powder components for granulation, to perform granulation, thereby obtaining granules (a fluidized-bed granulation method). Thereafter, sodium stearyl fumarate was added as a lubricant to the granules, and the resulting mixture was compressed (tableting pressure: 4 kN, 5 kN, or 6 kN) to obtain orally disintegrating tablets.

**Table 3**

| | Example 3-1 | Comparative Example 3-2 |
|---|---|---|
| Brexpiprazole | 2.0 mg | 2.0 mg |
| D-Mannitol | 66.1 mg | 70.1 mg |
| Crystalline cellulose (Ceolus PH-101) | 10 mg | 10 mg |
| Low-substituted hydroxypropyl cellulose (NBD-022) | 5 mg | 5 mg |
| Sucralose | 0.1 mg | 0.1 mg |
| Partially pregelatinized starch (PCS PC-10) | 5 mg | - |
| Pregelatinized starch (SWELSTAR WB-1) | - | 1 mg |
| Sodium stearyl fumarate | 1.8 mg | 1.8 mg |
| Total solid component amount | 90.0 mg | 90.0 mg |

The degree of pregelatinization of the partially pregelatinized starch (PCS PC-10; Asahi Kasei Corporation) used was 55 to 70%, and the degree of pregelatinization of the pregelatinized starch (SWELSTAR WB-1; Asahi Kasei Corporation) used was 90 to 100%. The partially pregelatinized starch used had a water-soluble component content of 3% or less.

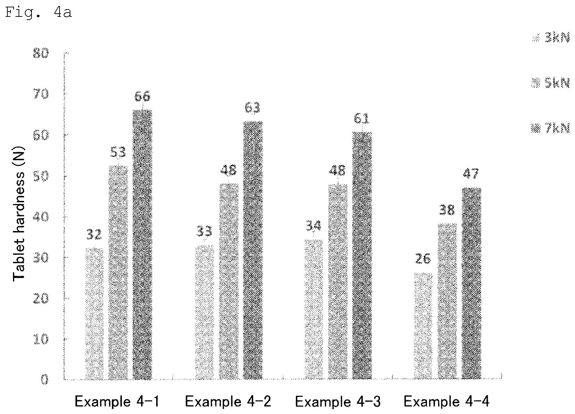

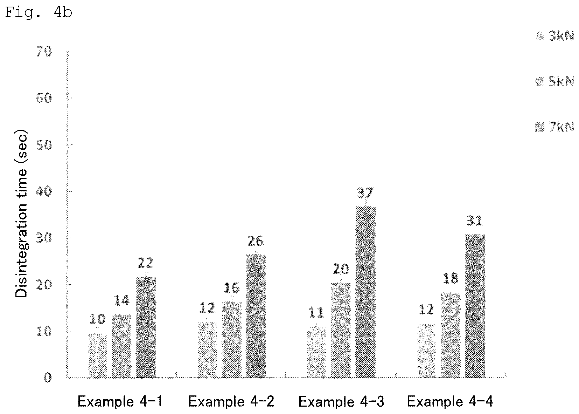

The disintegration property and hardness of each orally disintegrating tablet were measured. Fig. 4 shows the results. When the partially pregelatinized starch was used, a good disintegration property was exhibited while maintaining appropriate hardness. It was thus found that since both the disintegration property and the hardness can be maintained in a balanced manner, the use of the partially pregelatinized starch is more preferable. It can also be said that the range of tableting pressure conditions that simultaneously satisfy the preferred hardness and disintegration time was wider when the partially pregelatinized starch was used.

### Investigation of Lubricant

### Example 4-1

According to the formulation shown in Table 4, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). After sizing of the granules, a green pigment dispersion and a sweetener were added, followed by mixing, thereby obtaining a post-added mixed powder. The green pigment dispersion was obtained by subjecting Pigment Blend PB-1543 Green, which is a pigment component, to trituration with corn starch in an amount that is 5 times the weight of the pigment component. As the sweetener, sucralose was used. Thereafter, 0.9 mg of sodium stearyl fumarate (PRUV) was added as a lubricant to the post-added mixed powder, and the resulting mixture was compressed (tableting pressure: 3 kN, 5 kN, or 7 kN) to obtain orally disintegrating tablets.

### Example 4-2

According to the formulation shown in Table 4, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). After sizing of the granules, a green pigment dispersion and a sweetener were added, followed by mixing, thereby obtaining a post-added mixed powder. The green pigment dispersion was obtained by subjecting Pigment Blend PB-1543 Green, which is a pigment component, to trituration with corn starch in an amount that is 5 times the weight of the pigment component. As the sweetener, sucralose was used. Thereafter, 1.8 mg of sodium stearyl fumarate (PRUV) was added as a lubricant to the post-added mixed powder, and the resulting mixture was compressed (tableting pressure: 3 kN, 5 kN, or 7 kN) to obtain orally disintegrating tablets.

### Example 4-3

According to the formulation shown in Table 4, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). After sizing of the granules, a green pigment dispersion and a sweetener were added, followed by mixing, thereby obtaining a post-added mixed powder. The green pigment dispersion was obtained by subjecting Pigment Blend PB-1543 Green, which is a pigment component, to trituration with corn starch in an amount that is 5 times the weight of the pigment component. As the sweetener, sucralose was used. Thereafter, 3.6 mg of sodium stearyl fumarate (PRUV) was added as a lubricant to the post-added mixed powder, and the resulting mixture was compressed (tableting pressure: 3 kN, 5 kN, or 7 kN) to obtain orally disintegrating tablets.

### Example 4-4

According to the formulation shown in Table 4, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). After sizing of the granules, a green pigment dispersion and a sweetener were added, followed by mixing, thereby obtaining a post-added mixed powder. The green pigment dispersion was obtained by subjecting Pigment Blend PB-1543 Green, which is a pigment component, to trituration with corn starch in an amount that is 5 times the weight of the pigment component. As the sweetener, sucralose was used. Thereafter, 0.9 mg of magnesium stearate was added as a lubricant to the post-added mixed powder, and the resulting mixture was compressed (tableting pressure: 3 kN, 5 kN, or 7 kN) to obtain orally disintegrating tablets.

**Table 4**

| | Example 4-1 | Example 4-2 | Example 4-3 | Example 4-4 |
|---|---|---|---|---|
| Brexpiprazole | 2.0 mg | 2.0 mg | 2.0 mg | 2.0 mg |
| D-Mannitol | 65.6 mg | 65.6 mg | 65.6 mg | 65.6 mg |
| Crystalline cellulose (Ceolus PH-101) | 10.0 mg | 10.0 mg | 10.0 mg | 10.0 mg |
| Low-substituted hydroxypropyl cellulose (NBD-022) | 5.0 mg | 5.0 mg | 5.0 mg | 5.0 mg |
| Partially pregelatinized starch (PCS PC-10) | 5.0 mg | 5.0 mg | 5.0 mg | 5.0 mg |
| Pigment Blend PB-1543 Green | 0.09 mg | 0.09 mg | 0.09 mg | 0.09 mg |
| Corn starch | 0.45 mg | 0.45 mg | 0.45 mg | 0.45 mg |
| Sucralose | 0.1 mg | 0.1 mg | 0.1 mg | 0.1 mg |
| Sodium stearyl fumarate | 0.9 mg | 1.8 mg | 3.6 mg | - |
| Magnesium stearate | - | - | - | 0.9 mg |
| Total solid component amount | 89.1 mg | 90.0 mg | 91.8 mg | 89.1 mg |

Immediately after the tableting process, the hardness of each orally disintegrating tablet was measured, and the tablet was subjected to a disintegration test. Fig. 4a and Fig. 4b show the results. All of the orally disintegrating tablets had the hardness and disintegration property that were suitable for practical use. In particular, when sodium stearyl fumarate was used as a lubricant (e.g., Example 4-1 or 4-2), especially excellent orally disintegrating tablets with high hardness and a short disintegration time were obtained.

### Addition of Lubricant (Addition of External Lubricant) During Compressing

The production of the orally disintegrating tablets of Examples 4-1 to 4-4 by tableting was continued. In Examples 4-1 and 4-2, both of which use sodium stearyl fumarate as an internal lubricant, filming occurred when the production by tableting was continued for about 5 to 30 minutes. Filming also occurred in Example 4-3, although it was slight.

Filming is a phenomenon in which powder adheres to the surface of the punches of a rotary tablet press machine. When this happens, the tableting is performed with the punches having the powder adhered thereto; thus, the tables produced have a rough (coarse) surface. Moreover, if the tableting process is continued with filming occurring, the powder itself adhered to the punches agglomerates. If tablets are produced by tableting with such punches to which agglomerated powder adheres, a recess is formed on the tablet surface. Thus, each time filming occurs, the powder adhered to the punches should be removed, which is disadvantageous for mass production.

In addition, the tablet property (balance between the hardness and the disintegration property) was poor in Example 4-4, which uses magnesium stearate as an internal lubricant.

Thus, whether there was a method to prevent such filming was investigated. As a result, it was found that external lubrication method for compression, in which a lubricant is supplied externally, can prevent filming, as described below. More specifically, external lubrication method for compression is a method in which a small amount of a lubricant is forcedly charged and sprayed directly onto upper and lower punches, as well as onto dies.

Specifically, according to the formulation shown in Example 5-3 of Table 5a, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). After sizing of the granules, a yellow pigment dispersion, a blue pigment dispersion, and a sweetener were added thereto, followed by mixing, thereby obtaining a post-added mixed powder. The yellow pigment dispersion was obtained by subjecting yellow ferric oxide to trituration with corn starch in an amount that is 5 times the weight of yellow ferric oxide. The blue pigment dispersion was obtained by subjecting blue no. 2 aluminum lake to trituration with corn starch in an amount that is 5 times the weight of blue no. 2 aluminum lake. As the sweetener, sucralose was used. Thereafter, sodium stearyl fumarate was internally added as a lubricant. Further, 0.21 mg of magnesium stearate was added by spraying during compressing (tableting pressure: 3 to 8 kN) by using an external lubrication method for compression method to obtain orally disintegrating tablets. That is, sodium stearyl fumarate was used as an internal lubricant, and magnesium stearate was used as an external lubricant.

Table 5a also shows the compositions of Examples 4-2 and 4-4.

**Table 5a**

| | Example 4-2 | Example 4-4 | Example 5-3 |
|---|---|---|---|
| Brexpiprazole | 2.0 mg | 2.0 mg | 2.0 mg |
| D-Mannitol | 65.6 mg | 65.6 mg | 65.09 mg |
| Crystalline cellulose (Ceolus PH-101) | 10.0 mg | 10.0 mg | 10.0 mg |
| Low-substituted hydroxypropyl cellulose (NBD-022) | 5.0 mg | 5.0 mg | 5.0 mg |
| Partially pregelatinized starch (PCS PC-10) | 5.0 mg | 5.0 mg | 5.0 mg |
| Pigment Blend PB-1543 Green | 0.09 mg | 0.09 mg | - |
| Yellow ferric oxide | - | - | 0.27 mg |
| Blue no. 2 aluminum lake | - | - | 0.09 mg |
| Corn starch | 0.45 mg | 0.45 mg | 1.8 mg |
| Sucralose | 0.1 mg | 0.1 mg | 0.1 mg |
| Sodium stearyl fumarate (internal addition) | 1.8 mg | - | 0.45 mg |
| Magnesium stearate (internal addition) | - | 0.9 mg | - |
| Magnesium stearate (external addition) | - | - | 0.21 mg |
| Total solid component amount | 90.0 mg | 89.1 mg | 90.0 mg |

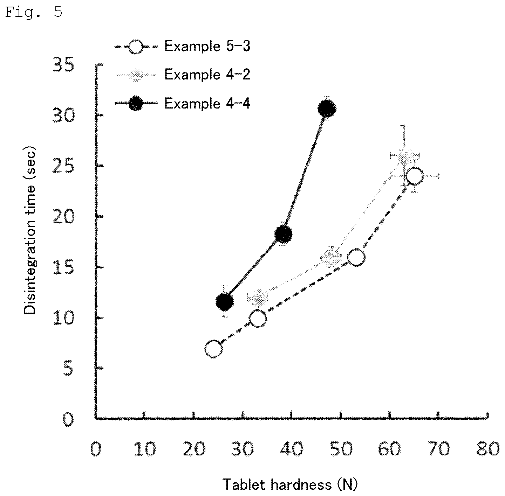

Table 5b and Fig. 5 show the results of investigating the tablet property (balance between the hardness and the disintegration property) and filming of the tablets of Examples 4-2, 4-4, and 5-3.

**Table 5b**

| Example | Internal addition | External addition | Tablet property | Filming (Photograph of punch surface) | |
|---|---|---|---|---|---|
| | | | | Before compressing | After compressing |
| 5 - 3 | Added (sodium stearyl fumarate) | Added (magnesium stearate) | Best | 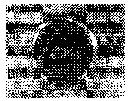 | 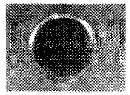 |
| 4 - 2 | Added (sodium stearyl fumarate) | Not added | Good | 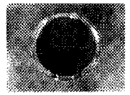 | 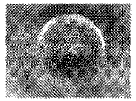 |
| 4 - 4 | Added (magnesium stearate) | Not added | Average | No data | No data |

Further, according to the formulations shown in Table 5c, orally disintegrating tablets (Examples 5-1, 5-2, and 5-4) were obtained by using an external lubrication method for compression in the same manner as in Example 5-3, except that 0.05, 0.10, or 0.38 mg of magnesium stearate was added by spraying during compressing (tableting pressure: 3 to 8 kN). Table 5c also shows the composition of Example 5-3.

**Table 5c**

| | Example 5-1 | Example 5-2 | Example 5-3 | Example 5-4 |
|---|---|---|---|---|
| Brexpiprazole | 2.0 mg | 2.0 mg | 2.0 mg | 2.0 mg |
| D-Mannitol | 65.09 mg | 65.09 mg | 65.09 mg | 65.09 mg |
| Crystalline cellulose (Ceolus PH-101) | 10.0 mg | 10.0 mg | 10.0 mg | 10.0 mg |
| Low-substituted hydroxypropyl cellulose (NBD-022) | 5.0 mg | 5.0 mg | 5.0 mg | 5.0 mg |
| Partially pregelatinized starch (PCS PC-10 ) | 5.0 mg | 5.0 mg | 5.0 mg | 5.0 mg |
| Yellow ferric oxide | 0.27 mg | 0.27 mg | 0.27 mg | 0.27 mg |
| Blue no. 2 aluminum lake | 0.09 mg | 0.09 mg | 0.09 mg | 0.09 mg |
| Corn starch | 1.8 mg | 1.8 mg | 1.8 mg | 1.8 mg |
| Sucralose | 0.1 mg | 0.1 mg | 0.1 mg | 0.1 mg |
| Sodium stearyl fumarate | 0.45 mg | 0.45 mg | 0.45 mg | 0.45 mg |
| Magnesium stearate | 0.05 mg | 0.10 mg | 0.21 mg | 0.38 mg |
| Total solid component amount | 89.9 mg | 89.9 mg | 90.0 mg | 90.2 mg |

Due to the difference in the amount of magnesium stearate added as an external lubricant, the total amounts of the solid components of the tablets of the Examples are slightly different.

No filming was observed in the production of any of the orally disintegrating tablets (Examples 5-1 to 5-4) by tableting. Further, all of the orally disintegrating tablets showed good hardness and a good disintegration time, indicating that both tabletability and oral disintegratability were achieved. Comparing the push-up pressure in the production process of the orally disintegrating tablets of each Example when tableting was performed at a tableting pressure of 6 kN, Example 5-1 showed a slightly high push-up pressure, but the other Examples showed low values. This indicates that continuous tableting was possible.

### Investigation of Particle Diameter of D-mannitol

According to the formulations shown in Table 6, orally disintegrating tablets of Examples 6-1 to 6-3 were produced using D-mannitol with different particle diameters.

### Example 6-1 to Example 6-3

According to the formulations shown in Table 6, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). As D-mannitol, Pearlitol 50C (Roquette) was used in Example 6-1, Pearlitol 160C (Roquette) was used in Example 6-2, and Pearlitol 300DC (Roquette) was used in Example 6-3. After sizing of the granules, a yellow pigment dispersion, a blue pigment dispersion, and a sweetener were added thereto, followed by mixing, thereby obtaining a post-added mixed powder. The yellow pigment dispersion was obtained by subjecting yellow ferric oxide to trituration with corn starch in an amount that is 5 times the weight of yellow ferric oxide. The blue pigment dispersion was obtained by subjecting blue no. 2 aluminum lake to trituration with corn starch in an amount that is 5 times the weight of blue no. 2 aluminum lake. As the sweetener, sucralose was used. Thereafter, sodium stearyl fumarate was internally added as a lubricant. Further, magnesium stearate was added by spraying during compressing (tableting pressure: 4 kN, 6 kN, or 8 kN) by using an external lubrication method for compression to obtain orally disintegrating tablets.

**Table 6**

| | Example 6-1 | Example 6-2 | Example 6-3 |
|---|---|---|---|
| Brexpiprazole | 2.0 mg | 2.0 mg | 2.0 mg |
| D-Mannitol (Pearlitol 50C) | 65.09 mg | - | - |
| D-Mannitol (Pearlitol 160C) | - | 65.09 mg | - |
| D-Mannitol (Pearlitol 300DC) | - | - | 65.09 mg |
| Crystalline cellulose (Ceolus PH-101) | 10.0 mg | 10.0 mg | 10.0 mg |
| Low-substituted hydroxypropyl cellulose (NBD-022) | 5.0 mg | 5.0 mg | 5.0 mg |
| Partially pregelatinized starch (PCS PC-10) | 5.0 mg | 5.0 mg | 5.0 mg |
| Yellow ferric oxide | 0.27 mg | 0.27 mg | 0.27 mg |
| Blue no. 2 aluminum lake | 0.09 mg | 0.09 mg | 0.09 mg |
| Corn starch | 1.8 mg | 1.8 mg | 1.8 mg |
| Sucralose | 0.1 mg | 0.1 mg | 0.1 mg |
| Sodium stearyl fumarate (internal lubricant) | 0.45 mg | 0.45 mg | 0.45 mg |
| Magnesium stearate (external lubricant) | 0.2 mg | 0.2 mg | 0.2 mg |
| Total solid component amount | 90.0 mg | 90.0 mg | 90.0 mg |

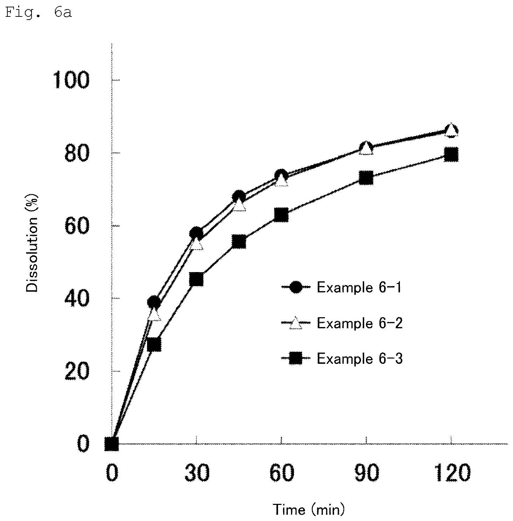

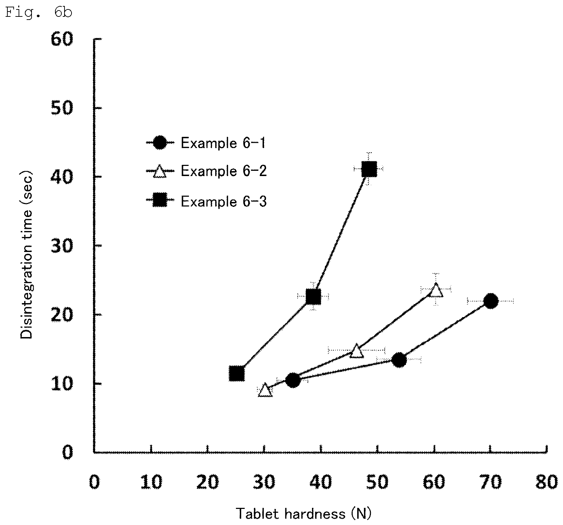

The dissolution property and the tablet property (balance between the hardness and the disintegration property) of the tablets of Example 6-1 (Pearlitol 50C, 50% particle diameter: 35 µm), Example 6-2 (Pearlitol 160C, 50% particle diameter: 70 µm), and Example 6-3 (Pearlitol 300DC, 50% particle diameter: 250 µm) were evaluated. Fig. 6a shows the results of the dissolution property. Fig. 6b shows the results of the tablet property. Examples 6-1 to 6-3 use D-mannitol with different particle diameters. The dissolution and tablet properties suitable for practical use were achieved when D-mannitol having each of the above particle diameters was used. Among them, the example using D-mannitol with a 50% particle diameter of 35 µm (Example 6-1) was found to be preferable. Although all of the tablets had an appearance that caused no problems in practical use, the tablets obtained using D-mannitol having a smaller particle diameter were found to be more preferable because the color tone on the tablet surface was even.

### Investigation of Crystal Shape of D-mannitol

According to the formulations shown in Table 7, orally disintegrating tablets of Examples 7-1 and 7-2 were produced.

### Example 7-1 and Example 7-2

According to the formulations shown in Table 7, a suspension of partially pregelatinized starch as a binder was added by spraying to brexpiprazole, D-mannitol, crystalline cellulose, and low-substituted hydroxypropyl cellulose, which are powder components for granulation, to perform granulation, thereby obtaining granules (a fluidized-bed granulation method). As D-mannitol, Pearlitol 50C (Roquette) was used in Example 7-1, and Parteck Delta M (Merck) was used in Example 7-2. After sizing of the granules, a yellow pigment dispersion, a blue pigment dispersion, and a sweetener were added thereto, followed by mixing, thereby obtaining a post-added mixed powder. The yellow pigment dispersion was obtained by subjecting yellow ferric oxide to trituration with corn starch in an amount that is 5 times the weight of yellow ferric oxide. The blue pigment dispersion was obtained by subjecting blue no. 2 aluminum lake to trituration with corn starch in an amount that is 5 times the weight of blue no. 2 aluminum lake. As the sweetener, sucralose was used. Thereafter, sodium stearyl fumarate was added as a lubricant to the post-added mixed powder, and the resulting mixture was compressed (tableting pressure: 8 kN) to obtain orally disintegrating tablets.

**Table 7**

| | Example 7-1 | Example 7-2 |
|---|---|---|
| Brexpiprazole | 2.0 mg | 2.0 mg |
| D-Mannitol (Pearlitol 50C) | 65.09 mg | - |
| D-Mannitol (Parteck Delta M) | - | 65.09 mg |
| Crystalline cellulose (Ceolus PH-101) | 10.0 mg | 10.0 mg |
| Low-substituted hydroxypropyl cellulose (NBD-022) | 5.0 mg | 5.0 mg |
| Partially pregelatinized starch (PCS PC-10) | 5.0 mg | 5.0 mg |
| Yellow ferric oxide | 0.27 mg | 0.27 mg |
| Blue no. 2 aluminum lake | 0.09 mg | 0.09 mg |
| Corn starch | 1.8 mg | 1.8 mg |
| Sucralose | 0.1 mg | 0.1 mg |
| Sodium stearyl fumarate | 1.8 mg | 1.8 mg |
| Total solid component amount | 91.15 mg | 91.15 mg |

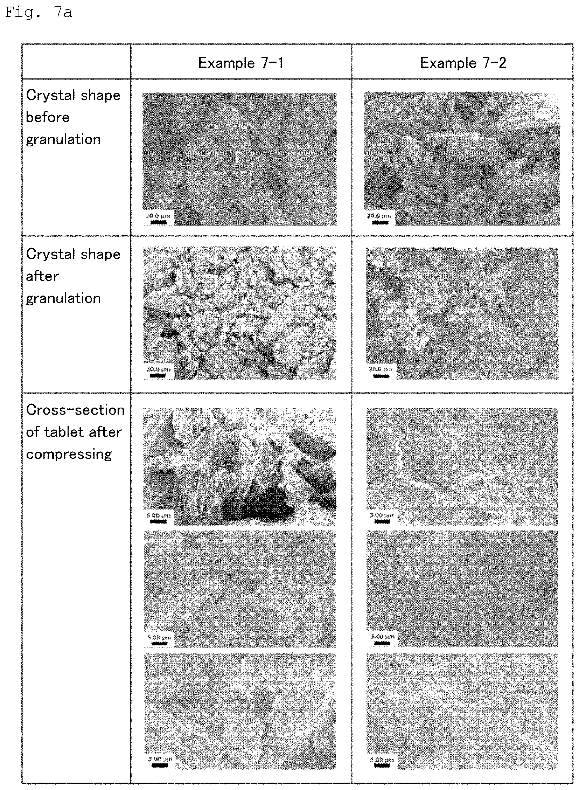

First, the crystal shape of D-mannitol before and after granulation and after compressing was evaluated for Example 7-1 (Pearlitol 50C) and Example 7-2 (Parteck Delta M). The crystal shape of D-mannitol was evaluated by observing the cross-section of the granules or tablets using an electron microscope (Real Surface View Microscope VE-7800 produced by KEYENCE) at 500x or 2000x magnification. Fig. 7a shows the results. Pearlitol 50C (Example 7-1) consistently showed a plate-like crystalline form before and after granulation. On the other hand, Parteck Delta M (Example 7-2) showed a plate-like crystalline form before granulation, but the crystalline form changed from a plate-like shape to a needle-like shape after granulation. When the subsequent production process was carried out using the obtained granules, it was possible to produce orally disintegrating tablets in both Examples. The difference between the plate-like and needle-like crystalline forms observed in the granules was also clearly confirmed in the cross-sections of the tablets obtained after compressing.

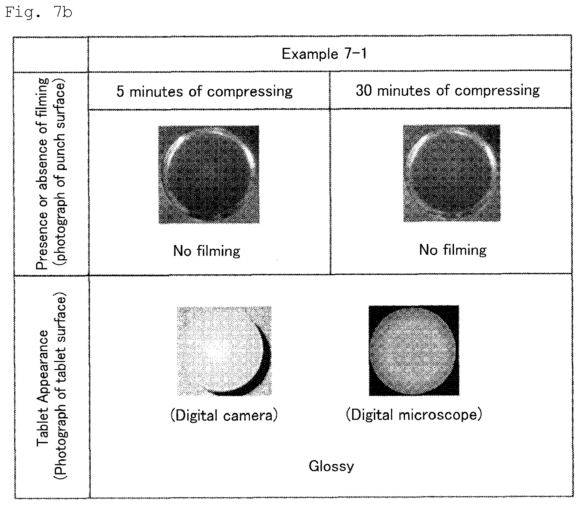

Next, the tabletability (presence or absence of filming) and tablet appearance were evaluated for Example 7-1 (Pearlitol 50C). The tablet appearance was evaluated using a digital camera and a digital microscope (VHX-500 produced by KEYENCE). Fig. 7b shows the results. When Pearlitol 50C was used as D-mannitol (Example 7-1), no filming was observed even 30 minutes after the start of compressing, indicating that a high filming inhibitory effect was exhibited. Moreover, the tablets obtained had a glossy surface.

## Claims

1. An orally disintegrating tablet comprising:
(A) brexpiprazole or a salt thereof;
(B) D-mannitol;
(C) partially pregelatinized starch; and
(D) a lubricant.

2. The orally disintegrating tablet according to claim 1, wherein the lubricant (D) comprises magnesium stearate and sodium stearyl fumarate.

3. The orally disintegrating tablet according to claim 1, wherein the lubricant (D) comprises (D1) an internal lubricant and (D2) an external lubricant.

4. The orally disintegrating tablet according to claim 3, wherein the internal lubricant (D1) comprises sodium stearyl fumarate, and the external lubricant (D2) comprises magnesium stearate.

5. The orally disintegrating tablet according to any one of claims 1 to 4, wherein the D-mannitol (B) has a 50% particle diameter of 10 µm to 100 µm, and the D-mannitol (B) is present in non-needle-like crystalline form in the tablet.

6. The orally disintegrating tablet according to any one of claims 1 to 5, wherein the partially pregelatinized starch (C) is partially pregelatinized starch having a water-soluble component content of 10% or less.

7. The orally disintegrating tablet according to any one of claims 1 to 6, further comprising (E) crystalline cellulose.

8. The orally disintegrating tablet according to claim 7, comprising the crystalline cellulose (E) in an amount of 5 to 15 wt%.

9. The orally disintegrating tablet according to any one of claims 1 to 8, further comprising (F) low-substituted hydroxypropyl cellulose.

10. The orally disintegrating tablet according to any one of claims 1 to 9, which has a tablet hardness of 15 N to 70 N in a diametrical direction thereof, as measured with a tablet hardness tester, and a disintegration time of 70 seconds or less, as measured by the test for immediate-release preparations (plain tablets) described in 6.09 Disintegration Test of the General Tests, Processes and Apparatus section of the Japanese Pharmacopoeia.

11. The orally disintegrating tablet according to any one of claims 1 to 10, which is for preventing or treating a central nervous system disease.

12. The orally disintegrating tablet according to claim 11, which is for preventing or treating a central nervous system disease selected from the group consisting of schizophrenia, treatment-resistant, refractory, or chronic schizophrenia, emotional disturbance, psychotic disorder, mood disorder, bipolar disorder, depression, endogenous depression, major depression, melancholic and treatment-resistant depression, dysthymic disorder, cyclothymic disorder, anxiety disorder, somatoform disorder, factitious disorder, dissociative disorder, sexual disorder, eating disorder, sleep disorder, adjustment disorder, substance-related disorder, anhedonia, delirium, cognitive impairment, cognitive impairment associated with neurodegenerative disease, cognitive impairment caused by neurodegenerative disease, cognitive impairment in schizophrenia, cognitive impairment caused by treatment-resistant, refractory, or chronic schizophrenia, vomiting, motion sickness, obesity, migraine, pain, mental retardation, autistic disorder, Tourette's disorder, tic disorder, attention deficit hyperactivity disorder, conduct disorder, Down's syndrome, impulsive symptoms associated with dementia, and borderline personality disorder.

13. An orally disintegrating tablet comprising:
(A) brexpiprazole or a salt thereof;
(B) D-mannitol;
(C) partially pregelatinized starch; and
(D) a lubricant,
the orally disintegrating tablet being produced by external lubrication method for compression.

14. A method for producing an orally disintegrating tablet comprising (A) brexpiprazole or a salt thereof, (B) D-mannitol, (C) partially pregelatinized starch, and (D) a lubricant, the lubricant (D) comprising (D1) an internal lubricant and (D2) an external lubricant, the method comprising the steps of:
mixing (A) brexpiprazole or a salt thereof, (B) D-mannitol, (C) partially pregelatinized starch, and (D1) an internal lubricant; and
compressing the mixture,
wherein in the compression step, (D2) an external lubricant is added by spraying.
